# EUROPEAN PATENT APPLICATION

(11) **EP 3 943 495 A1**
(43) Date of publication of application: **26.01.2022**
(21) Application number: 20187785.9
(22) Date of filing: 24.07.2020
(51) Int. Cl.: C07D 487/04, A61P 17/06, A61P 19/02, A61P 37/02, A61K 31/5025

(54) **SMALL MOLECULE MODULATORS OF IL-17**

(71) Applicant: Leo Pharma A/S, 2750 Ballerup (DK)
(72) Inventor: ANDREWS, Mark David, 2750 Ballerup (DK); SEITZBERG, Jimmi Gerner, 2750 Ballerup (DK); DACK, Kevin Neil, 2750 Ballerup (DK)
(74) Representative: Leo Pharma A/S

(57) **Abstract**

The present invention relates to a compound according to formula I and pharmaceutically acceptable salts, hydrates, or solvates thereof. The invention further relates to said compounds for use in therapy, for example for the treatment of diseases such as autoimmune diseases, psoriasis, ankylosing spondylitis, spondyloarthritis or psoriatic arthritis, and to pharmaceutical compositions comprising said compounds.

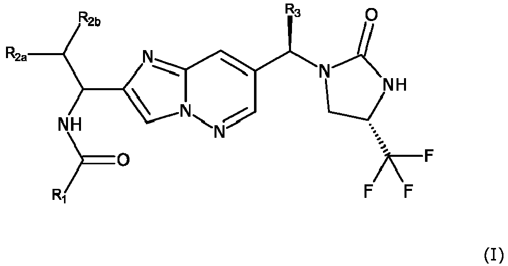

## Description

### FIELD OF THE INVENTION

This invention relates to novel imidazo[1,2-b]pyrazines and derivatives thereof, to said compounds for use in therapy and to pharmaceutical compositions comprising said compounds.

### BACKGROUND OF THE INVENTION

IL-17 (also known as IL-17A or CTLA8) is a pro-inflammatory cytokine involved in antimicrobial defense at epithelial surfaces. IL-17 is comprised of two covalently joined IL-17A subunits (IL-17AA) with an approximate mass of 32 kDa, and signals through a receptor comprising IL17RA and IL17RC subunits. This receptor is predominantly expressed in epithelial and mesenchymal cells. The IL17RA/IL17RC receptor is also used by IL-17 variants IL-17AF and IL-17FF, which both are successively weaker, partial agonists on this receptor (Monin, L., Gaffen, S.L.; 2018, Cold Spring Harb. Perspect. Biol. 10. doi:10.1101/cshperspect.a028522). Crucial for signaling is the assembly of signaling complexes containing the multifunctional protein ACT1/CIKS, which in turn can recruit TRAF and other proteins.

Via these signaling complexes IL-17 induces cytokines, chemokines, antimicrobial peptides and growth factors via activation of transcription factor NFkB or via MAP kinase-dependent pathways (e.g. IL-6, IL-8, CXCL1, CXCL2, CXCL5, CCL20, G-CSF, BD4) and stabilizes the mRNAs of certain inflammatory cytokines, such as CXCL1. This leads to amplification of their effects. Further, IL-17 acts in concert with IL-1beta, IL-22 and IFNgamma (Amatya, N. et al., Trends in Immunology, 2017, 38, 310-322. doi:10.1016/j.it.2017.01.006; Onishi, R.M., Gaffen, S.L. Immunology, 2010, 129, 311-321. doi:10.1111/j.1365-2567.2009.03240.x).

IL-17 is secreted by a variety of immune cells, such as Th17 helper cells, Tc17 cytotoxic cells, ILC3 innate cells, NKT cells, TCRbeta+ natural T cells and gamma-deltaT-cells (Monin, L., Gaffen, S.L.; 2018, Cold Spring Harb. Perspect. Biol. 10. doi:10.1101/cshperspect.a028522). Increased, disease-provoking levels of IL-17 are observed in several autoimmune diseases, such as psoriasis, ankylosing spondylitis, spondyloarthritis and psoriatic arthritis. Other diseases where deregulation of IL-17 is observed are rheumatoid arthritis, systemic lupus erythematosus, asthma, inflammatory bowel disease, autoimmune uveitis, multiple sclerosis and certain cancers (Gaffen, S.L. et al., Nat Rev Immunol., 2014, 14, 585-600. doi:10.1038/nri3707; Monin, L., Gaffen, S.L.; 2018, Cold Spring Harb. Perspect. Biol. 10. doi:10.1101/cshperspect.a028522). Hence, IL-17 is a significant therapeutic target. Therapeutic, neutralizing antibodies against IL-17A (Secukinumab, Ixekizumab) or receptor IL17RA (Brodalumab) have shown high efficacy in the treatment of psoriasis, ankylosing spondylitis and psoriatic arthritis. These antibodies have long half-lives in the body.

Although various antibodies against IL-17A or IL-17RA are approved, only very few small molecule orally available modulators of IL-17 are known.

WO2013116682 discloses Macrocyclic Compounds for Modulating IL-17;

WO2014066726 discloses Compounds for Modulating IL-17;

WO2018229079 discloses Compounds for Modulating IL-17;

WO2019223718 discloses Compounds for Modulating IL-17;

WO2019138017 discloses Compounds for Modulating IL-17;

WO2020011731 discloses Compounds for Modulating IL-17;

WO2020120140 discloses Compounds for Modulating IL-17;

WO2020120141 discloses Compounds for Modulating IL-17

WO2020146194 discloses IL-17A inhibitors.

Scientific Reports (2016) 6, 30859 discloses Macrocyclic IL-17A Antagonists.

Leslie Dakin, 12th Swiss Course on Medicinal Chemistry, Leysin, October 09-14, 2016 discloses 'Hit Identification, binding site elucidation and structure guided design of novel macrocyclic IL-17A antagonists'.

Orally available, highly efficacious small molecule IL-17 modulators which bind to IL-17 to decrease its functional ability to activate the IL-17 receptor complex may have a number of advantages compared to monoclonal antibodies. Oral administration and flexible treatment regimen may be two significant aspects in favour of patient convenience and the compounds may exhibit improved safety due to the possibility of faster withdrawal of the drug should adverse events occur.

Therefore, there is a continuous need to develop small molecule modulators of IL-17, particularly small molecules suitable for oral administration.

In addition, some patients may be treated by topical application of small molecule modulators of IL-17. This can be particularly suitable for patients with skin lesions that are readily accessible and limited in body surface area. Topical treatment may also be prescribed for certain patients who could benefit from avoiding systemic modulation of the IL-17 pathway, for example when undergoing treatment for infections or gastrointestinal problems.

### SUMMARY OF THE INVENTION

The inventors have surprisingly found that novel compounds of the present invention exhibit modulating effect on the IL-17 signalling pathway.

Compounds of the present invention may have advantageous properties such as high metabolic stability and/or membrane permeability properties that make them suitable for oral administration. Other compounds of the present invention may have advantageous properties for local topical therapy, such as high skin permeability and high metabolic instability.

Compounds of the present invention may be beneficial in preventing, treating or ameliorating a variety of diseases which involve up-regulation or de-regulation of IL-17, such as for example psoriasis, ankylosing spondylitis and psoriatic arthritis.

Accordingly, the present invention relates to a compound according to formula (I) 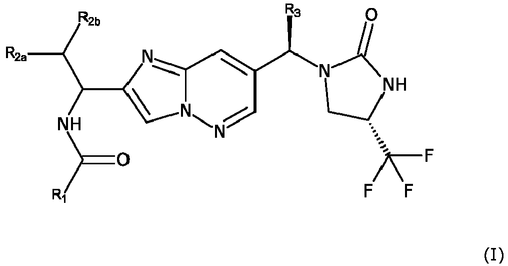
wherein R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from Rₐ;
Rₐ is (C₁-C₆)alkyl or (C₃-C₆)cycloalkyl, wherein said (C₁-C₆)alkyl and (C₃-C₆)cycloalkyl, is optionally substituted with one or more substituents independently selected from deuterium, halogen, hydroxy, and cyano;
R₂ₐ and R_{2b} are independently cyclopropyl and cyclobutyl or R₂ₐ and R_{2b} together form a 4-methyl-cyclohexyl ring;
R₃ is hydrogen or methoxymethyl;
or pharmaceutically acceptable salts, hydrates and solvates thereof.

In one embodiment the present invention relates to compounds of formula (Ia) 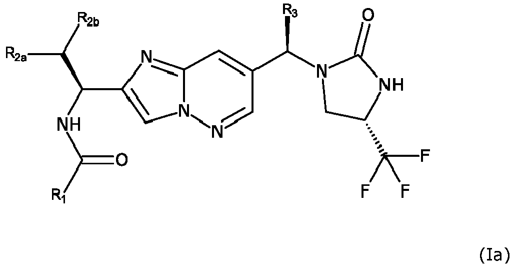 wherein R₁, R₂ₐ, R_{2b} and R₃ are as defined above or pharmaceutically acceptable salts, hydrates and solvates thereof.

In another aspect, the invention relates to a pharmaceutical composition comprising a compound of general formula (I) as defined herein together with a pharmaceutically acceptable vehicle or excipient or pharmaceutically acceptable carrier(s), optionally together with one or more other therapeutically active compound(s).

In yet another aspect, the invention relates to the use of a compound according to formula I as defined herein for use in therapy, for example for use in treatment of a disease, disorder or condition, which disease, disorder or condition is responsive of modulation of IL-17, for example for use in treatment of autoimmune diseases.

### DETAILED DESCRIPTION OF THE INVENTION

### Definitions

The term "(Cₐ-C_{b})alkyl" is intended to indicate a hydrocarbon radical obtained when one hydrogen atom is removed from a branched or linear hydrocarbon. Said alkyl comprises (a-b) carbon atoms, such as 1-6, such as 1-4, such as 1-3, such as 2-3 or such as 1-2 carbon atoms. The term includes the subclasses normal alkyl (*n*-alkyl), secondary and tertiary alkyl, such as methyl, ethyl, *n*-propyl, isopropyl, *n*-butyl, isobutyl, *sec*.-butyl, *tert*.-butyl, *n*-pentyl, isopentyl, neopentyl, *n*-hexyl and isohexyl.

The term "(Cₐ-C_{b})alkoxy" are intended to indicate a radical of the formula -OR', wherein R' is (Cₐ-C_{b})alkyl as indicated herein, wherein the (Cₐ-C_{b})alkyl group is appended to the parent molecular moiety through an oxygen atom, e.g. methoxy (-OCH₃), ethoxy (-OCH₂CH₃), n-propoxy, isopropoxy, butoxy, tert-butoxy, and the like.

The term "cyano" is intended to indicate a -CN group attached to the parent molecular moiety through the carbon atom.

The term "(Cₐ-C_{b})cycloalkyl" is intended to indicate a saturated (Cₐ-C_{b})cycloalkane hydrocarbon radical, including polycyclic radicals such as bicyclic or tricyclic radicals, including spirocyclic radicals, comprising a-b carbon atoms, such as 3-10 carbon atoms, such as 3-8 carbon atoms, such as 3-7 carbon atoms, such as 3-6 carbon atoms, such as 3-5 carbon atoms or such as 3-4 carbon atoms, e.g. cyclopropyl, cyclobutyl, cyclopentyl, cyclohexyl, spiro[2.3]hexanyl and bicyclo[3,1,0]hexanyl.

The term "halo(Cₐ-C_{b})alkyl" is intended to indicate an (Cₐ-C_{b})alkyl group as defined herein substituted with one or more halogen atoms as defined herein, e.g. fluoro or chloro, such as difluoromethyl or trifluoromethyl.

The term "halogen" is intended to indicate a substituent from the 7^{th} main group of the periodic table, such as fluoro, chloro and bromo.

The term "hydrocarbon radical" is intended to indicate a radical containing only hydrogen and carbon atoms, it may contain one or more double and/or triple carbon-carbon bonds, and it may comprise cyclic moieties in combination with branched or linear moieties. Said hydrocarbon comprises 1-6 carbon atoms, e.g. 1-5, e.g. 1-4, e.g. 1-3, e.g. 1-2 carbon atoms. The term includes alkyl and cycloalkyl as indicated herein.

The term "hydroxy (Cₐ-C_{b})alkyl" is intended to indicate an (Cₐ-C_{b})alkyl group as defined above substituted with one or more hydroxy, e.g. hydroxymethyl, hydroxyethyl, hydroxypropyl.

The term "cyano (Cₐ-C_{b})alkyl" is intended to indicate an (Cₐ-C_{b})alkyl group as defined above substituted with one or more cyano, e.g. cyanomethyl, cyanoethyl and cyanopropyl.

If substituents are described as being independently selected from a group, each substituent is selected independent of the other. Each substituent may therefore be identical or different from the other substituent(s).

The term "optionally substituted" means "unsubstituted or substituted", and therefore the general formulas described herein encompasses compounds containing the specified optional substituent(s) as well as compounds that do not contain the optional substituent(s).

As used herein whenever a molecular drawing of a substituent contains an arrow - the arrow indicates the bond attaching the substituent to the rest of the molecule.

The term "pharmaceutically acceptable salt" is intended to indicate salts prepared by reacting a compound of formula I, which comprise a basic moiety, with a suitable inorganic or organic acid, such as hydrochloric, hydrobromic, hydroiodic, sulfuric, nitric, phosphoric, formic, acetic, 2,2-dichloroacetic, adipic, ascorbic, L-aspartic, L-glutamic, galactaric, lactic, maleic, L-malic, phthalic, citric, propionic, benzoic, glutaric, gluconic, D-glucuronic, methanesulfonic, salicylic, succinic, malonic, tartaric, benzenesulfonic, ethane-1,2-disulfonic, 2-hydroxy ethanesulfonic acid, toluenesulfonic, sulfamic or fumaric acid. Pharmaceutically acceptable salts of compounds of formula I comprising an acidic moiety may also be prepared by reaction with a suitable base such as sodium hydroxide, potassium hydroxide, magnesium hydroxide, calcium hydroxide, zinc hydroxide, barium hydroxide, ammonia or the like, or suitable non-toxic amines, such as lower alkylamines (such as diethylamine, tetraalkylammonium hydroxide), hydroxy-lower alkylamines (such as diethanolamine, 2-(diethylamino)-ethanol, ethanolamine, triethanolamine, tromethamine, deanol), cycloalkylamines, ethylene diamine, or benzylamines, (such as benethamine and benzathine), betaine, choline hydroxide, N-methyl-glucamine, hydrabamine, 1H-imidazole, 4-(2-hydroxyethyl)-morpholine, piperazine, 1-(2-hydroxyethyl)-pyrrolidine, L-arginine or L-lysine. Further examples of pharmaceutical acceptable salts are listed in Berge, S.M.; J. Pharm. Sci.; (1977), 66(1), 1-19, and Stahl, P.H. and in Wermuth, C.G, Handbook of Pharmaceutical Salts, Properties, Selection and Use, 2nd Edition, Wiley-VCH, 2011 both of which are incorporated herein by reference.

The term 'prodrug' is intended to indicate compounds which are drug-precursors which, upon administration, are converted to the parent drug *in vivo* by enzymatic and/or chemical reactions. Generally, the pro-drug is less biologically active than its parent drug. The prodrug may have improved physical-chemical properties compared to the parent drug, such as improved aqueous solubility, thereby facilitating the absorption and consequently the bioavailability of the parent compound upon administration.

The term 'parent drug' or 'parent compound' is intended to indicate the biologically active compound which is released from the prodrug via enzymatic and/or chemical processes following administration of the prodrug. The parent drug is frequently the starting material for the preparation of the corresponding prodrug.

Examples of prodrugs according to the invention are prodrugs that are attached to a nitrogen or oxygen of the parent molecule.

The term "solvate" is intended to indicate a species formed by interaction between a compound, e.g. a compound of formula I, and a solvent, e.g. alcohol, glycerol or water, wherein said species are in a crystalline form. When water is the solvent, said species is referred to as a hydrate.

The term "or pharmaceutically acceptable salts, hydrates or solvates thereof" includes compound of formula (I) and hydrates or solvates thereof, and pharmaceutically acceptable salts of the compounds of formula(I) as well as hydrates or solvates thereof.

The term "treatment" as used herein means the management and care of a patient for the purpose of combating a disease, disorder or condition. The term is intended to include the delaying of the progression of the disease, disorder or condition, the amelioration, alleviation or relief of symptoms and complications, and/or the cure or elimination of the disease, disorder or condition. The term may also include prevention of the condition, wherein prevention is to be understood as the management and care of a patient for the purpose of combating the disease, condition or disorder and includes the administration of the active compounds to prevent the onset of the symptoms or complications. Nonetheless, prophylactic (preventive) and therapeutic (curative) treatments are two separate aspects.

All references, including publications, patent applications and patents, cited herein are hereby incorporated by reference in their entirety and to the same extent as if each reference were individually and specifically indicated to be incorporated by reference, regardless of any separately provided incorporation of particular documents made elsewhere herein.

In one embodiment, the invention relates to a compound of formula (I) or (Ia) as defined above, wherein R₂ₐ and R_{2b} are both cyclopropyl.

In one embodiment, the invention relates to a compound of formula(I) or (Ia) as defined above, wherein R₂ₐ and R_{2b} together form 4-methyl-cyclohexyl.

In another embodiment, the invention relates to a compound of formula (I) or (Ia) as defined above, wherein R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from (C₁-C₆)alkyl, (C₃-C₆)cycloalkyl and fluoro (C₁-C₆)alkyl.

In one embodiment, the invention relates to a compound of formula (I) or (Ia) as defined above, wherein R₁ is optionally substituted 1,2,3-triazole.

In one embodiment, the invention relates to a compound of formula (I) or (Ia) as defined above, wherein R₁ is optionally substituted pyrazole.

In one embodiment, the invention relates to a compound of formula (I) or (Ia) as defined above, wherein, R₁ is optionally substituted isoxazole.

In one embodiment, the invention relates to a compound of formula (I) or (Ia) as defined above, wherein R₁ is optionally substituted oxadiazole.

In one embodiment the invention relates to a compound of formula (I) or (Ia) as defined above, wherein R₁ is selected from 1,2,3-triazole, pyrazole, isoxazole or oxadiazole, wherein the 1,2,3-triazole, pyrazole, isoxazole or oxadiazole may optionally be substituted with one substituent selected from methyl, ethyl, isopropyl, cyclopropyl and trifluoromethyl.

In one embodiment, the invention relates to a compound of formula (I) selected from N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-4-methyl-1,2,5-oxadiazole-3-carboxamide;
4-cyclopropyl-N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-1,2,5-oxadiazole-3-carboxamide;
N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-2-isopropyl-pyrazole-3-carboxamide;
N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-3-ethyl-isoxazole-4-carboxamide;
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-4-methyl-1,2,5-oxadiazole-3-carboxamide;
4-cyclopropyl-N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-1,2,5-oxadiazole-3-carboxamide;
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-2-isopropyl-pyrazole-3-carboxamide;
N-[(S)-[7-[(1S)-2-methoxy-1-[(4S)-2-oxo-4-(trifluoromethyl)imidazolidin-1-yl]ethyl]imidazo[1,2-b]pyridazin-2-yl]-(4-methylcyclohexyl)methyl]-4-methyl-1,2,5-oxadiazole-3-carboxamide;
4-methyl-N-[(S)-(4-methylcyclohexyl)-[7-[[(4S)-2-oxo-4-(trifluoromethyl)imidazolidin-1-yl]methyl]imidazo[1,2-b]pyridazin-2-yl]methyl]-1,2,5-oxadiazole-3-carboxamide; and
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-3-ethyl-isoxazole-4-carboxamide
or pharmaceutically acceptable salts, hydrates and solvates thereof.

In one or more embodiments of the present invention, the compounds of general formula I have an (EC₅₀) value in an IL-8 release assay of less than 1 micromolar, or of less than 100 nanomolar, preferably less than 10 nanomolar.

The compounds of formula I may be obtained in crystalline form either directly by concentration from an organic solvent or by crystallisation or recrystallisation from an organic solvent or mixture of said solvent and a cosolvent that may be organic or inorganic, such as water. The crystals may be isolated in essentially solvent-free form or as a solvate, such as a hydrate. The invention covers all crystalline forms, such as polymorphs and pseudopolymorphs, and also mixtures thereof.

Compounds of formula I comprise asymmetrically substituted (chiral) carbon atoms which give rise to the existence of isomeric forms, e.g. enantiomers and possibly diastereomers. The present invention relates to all such isomers, either in optically pure form or as mixtures thereof (e.g. racemic mixtures or partially purified optical mixtures). Pure stereoisomeric forms of the compounds and the intermediates of this invention may be obtained by the application of procedures known in the art. The various isomeric forms may be separated by physical separation methods such as selective crystallization and chromatographic techniques, e.g. high-pressure liquid chromatography using chiral stationary phases. Enantiomers may be separated from each other by selective crystallization of their diastereomeric salts which may be formed with optically active amines, or with optically active acids. Optically purified compounds may subsequently be liberated from said purified diastereomeric salts. Enantiomers may also be resolved by the formation of diastereomeric derivatives. Alternatively, enantiomers may be separated by chromatographic techniques using chiral stationary phases. Pure stereoisomeric forms may also be derived from the corresponding pure stereoisomeric forms of the appropriate starting materials, provided that the reaction occur stereoselectively or stereospecifically. Preferably, if a specific stereoisomer is desired, said compound will be synthesized by stereoselective or stereospecific methods of preparation. These methods will advantageously employ chiral pure starting materials.

Furthermore, when a double bond or a fully or partially saturated ring system is present in the molecule geometric isomers may be formed. Any geometric isomer, as separated, pure or partially purified geometric isomers or mixtures thereof are included within the scope of the invention.

The formula (I) and (Ia) includes all tautomers and all mixtures thereof.

In the compounds of general Formula I, the atoms may exhibit their natural isotopic abundances, or one or more of the atoms may be artificially enriched in a particular isotope having the same atomic number, but an atomic mass or mass number different from the atomic mass or mass number found in nature. The present invention includes all suitable isotopic variations of the compounds of general Formula I. For example, different isotopic forms of hydrogen include ¹H, ²H and ³H, different isotopic forms of carbon include ¹²C, ¹³C and ¹⁴C and different isotopic forms of nitrogen include ¹⁴N and ¹⁵N. Enriching for deuterium (²H) may for example increase in-vivo half-life or reduce dosage regiments, or may provide a compound useful as a standard for characterization of biological samples. Isotopically enriched compounds within general formula I can be prepared by conventional techniques well known to a person skilled in the art or by processes analogous to those described in the general procedures and examples herein using appropriate isotopically enriched reagents and/or intermediates.

Some compounds have lower aqueous solubility which may affect the absorption and consequently the bioavailability of the compounds. Such compounds may advantageously be administered in the form of prodrugs improving the aqueous solubility of the parent compound. Such prodrugs which, upon administration, are converted to their parent compounds may be less active *in vitro* compared to their parent compounds, but because of the improved aqueous solubility, facilitating the absorption and consequently the bioavailability of the parent compounds upon administration, such prodrugs have improved *in vivo* activity compared to their parent compounds.

Prodrugs of the compounds of formula (I) form part of the invention claimed.

Solvates and hydrates form part of the invention claimed.

The compounds of the present invention may be useful for preventing, treating or ameliorating any of the following diseases: psoriasis, ankylosing spondylitis, spondyloarthritis or psoriatic arthritis, lichen planus, lupus nephritis, Sjögren's syndrome, acne, vitiligo, alopecia areata, ichthyosis, acute and chronic liver diseases, gout, osteoarthritis, SLE (besides LN and DLE), multiple sclerosis, plaque psoriasis, pustular psoriasis, rheumatoid arthritis, pityriasis rubra pilaris, pyoderma gangrenosum, hidradenitis suppurativa, discoid lupus erythematosus, Papulopustolar rosacea, atopic dermatitis, Ichthyosis, bullous pemphigoid, scleroderma, rheumatoid arthritis, tendinopathy, chronic wounds and cancer.

In an embodiment the invention relates to the use of a compound of general formula (I) as defined above, in the manufacture of a medicament for the prophylaxis, treatment or amelioration of any of the following diseases: psoriasis, ankylosing spondylitis, spondyloarthritis or psoriatic arthritis, lichen planus, lupus nephritis, Sjögren's syndrome, acne, vitiligo, alopecia areata, ichthyosis, acute and chronic liver diseases, gout, osteoarthritis, SLE (besides LN and DLE), multiple sclerosis, plaque psoriasis, pustular psoriasis, rheumatoid arthritis, pityriasis rubra pilaris, pyoderma gangrenosum, hidradenitis suppurativa, discoid lupus erythematosus, Papulopustolar rosacea, atopic dermatitis, Ichthyosis, bullous pemphigoid, scleroderma, rheumatoid arthritis, tendinopathy, chronic wounds and cancer.

In an embodiment the invention relates to the use of a compound of general formula (I) as defined above, in the manufacture of a medicament for the prophylaxis, treatment or amelioration of autoimmune diseases, such as psoriasis, ankylosing spondylitis, spondyloarthritis or psoriatic arthritis.

In an embodiment the invention relates to a method of preventing, treating or ameliorating autoimmune diseases, such as psoriatic arthritis, lichen planus, lupus nephritis, Sjögren's syndrome, acne, vitiligo, alopecia areata, ichthyosis, acute and chronic liver diseases, gout, osteoarthritis, SLE (besides LN and DLE), multiple sclerosis, plaque psoriasis, pustular psoriasis, rheumatoid arthritis, pityriasis rubra pilaris, pyoderma gangrenosum, hidradenitis suppurativa, discoid lupus erythematosus, Papulopustolar rosacea, atopic dermatitis, Ichthyosis, bullous pemphigoid, scleroderma, rheumatoid arthritis, tendinopathy, chronic wounds and cancer, the method comprising administering to a person suffering from at least one of said diseases an effective amount of one or more compounds according to general formula (I), optionally together with a pharmaceutically acceptable carrier or one or more excipients, optionally in combination with other therapeutically active compounds.

In an embodiment the invention relates to a method of preventing, treating or ameliorating autoimmune diseases, such as psoriasis, ankylosing spondylitis, spondyloarthritis or psoriatic arthritis, the method comprising administering to a person suffering from at least one of said diseases an effective amount of one or more compounds to according to general formula (I), optionally together with a pharmaceutically acceptable carrier or one or more excipients, optionally in combination with other therapeutically active compounds.

Besides being useful for human treatment, the compounds of the present invention may also be useful for veterinary treatment of animals including mammals such as horses, cattle, sheep, pigs, dogs, and cats.

### Pharmaceutical Compositions of the Invention

For use in therapy, compounds of the present invention are typically in the form of a pharmaceutical composition. The invention therefore relates to a pharmaceutical composition comprising a compound of formula I, optionally together with one or more other therapeutically active compound(s), together with a pharmaceutically acceptable excipient, vehicle or carrier(s). The excipient must be "acceptable" in the sense of being compatible with the other ingredients of the composition and not deleterious to the recipient thereof.

Conveniently, the active ingredient comprises from 0.0001-99.9% by weight of the formulation.

In the form of a dosage unit, the compound may be administered one or more times a day at appropriate intervals, always depending, however, on the condition of the patient, and in accordance with the prescription made by the medical practitioner. Conveniently, a dosage unit of a formulation contain between 0.001 mg and 1000 mg, preferably between 0.01 mg and 300 mg of a compound of formula I.

A suitable dosage of the compound of the invention will depend, inter alia, on the age and condition of the patient, the severity of the disease to be treated and other factors well known to the practising physician. The compound may be administered either orally, parenterally, topically, transdermally or interdermally and other routes according to different dosing schedules, e.g. daily, weekly or with monthly intervals. In general a single dose will be in the range from 0.001 to 400 mg/kg body weight.

If the treatment involves administration of another therapeutically active compound it is recommended to consult Goodman & Gilman's The Pharmacological Basis of Therapeutics, 9th Ed., J.G. Hardman and L.E. Limbird (Eds.), McGraw-Hill 1995, for useful dosages of said compounds.

The administration of a compound of the present invention with one or more other active compounds may be either concomitantly or sequentially.

The formulations include e.g. those in a form suitable for oral, rectal, parenteral transdermal, intradermal, ophthalmic, topical, nasal, sublingual or buccal administration.

The formulations may conveniently be presented in dosage unit form and may be prepared by but not restricted to any of the methods well known in the art of pharmacy, e.g. as disclosed in Remington, The Science and Practice of Pharmacy, 21ed ed., 2005. All methods include the step of bringing the active ingredient into association with the carrier, which constitutes one or more accessory ingredients. In general, the formulations are prepared by uniformly and intimately bringing the active ingredient into association with a liquid carrier, semisolid carrier or a finely divided solid carrier or combinations of these, and then, if necessary, shaping the product into the desired formulation.

Formulations of the present invention suitable for oral and buccal administration may be in the form of discrete units as capsules, sachets, tablets, chewing gum or lozenges, each containing a predetermined amount of the active ingredient.

A tablet may be made by compressing, moulding or freeze drying the active ingredient optionally with one or more accessory ingredients. Compressed tablets may be prepared by compressing, in a suitable machine, the active ingredient(s) in a free-flowing form; for example, with a lubricant; a disintegrating agent or a dispersing agent. Moulded tablets may be made by moulding, in a suitable machine, a mixture of the powdered active ingredient and suitable carrier. Freeze dried tablets may be formed in a freeze-dryer from a solution of the drug substance.

Formulations suitable for parenteral administration conveniently comprise a sterile oily or aqueous preparation of the active ingredients, which is preferably isotonic with the blood of the recipient, e.g. isotonic saline, isotonic glucose solution or buffer solution. Liposomal formulations are also suitable for parenteral administration.

Transdermal formulations may be in the form of a plaster, patch, microneedles, liposomal or nanoparticulate delivery systems or other cutaneous formulations applied to the skin.

Formulations suitable for ophthalmic administration may be in the form of a sterile aqueous preparation of the active ingredients. Liposomal formulations or biodegradable polymer systems may also be used to present the active ingredient for ophthalmic administration.

Formulations suitable for topical, such as dermal, intradermal or ophthalmic administration include liquid or semi-solid preparations, solutions or suspensions.

Formulations suitable for nasal or buccal administration include powder, self-propelling and spray formulations, such as aerosols and atomisers.

### METHODS OF PREPARATION

The compounds of the present invention can be prepared in a number of ways well known to those skilled in the art of synthesis. The compounds of the invention could for example be prepared using the reactions and techniques outlined below together with methods known in the art of synthetic organic chemistry, or variations thereof as appreciated by those skilled in the art. Preferred methods include, but are not limited to, those described below. The reactions are carried out in solvents appropriate to the reagents and materials employed and suitable for the transformations being affected. Also, in the synthetic methods described below, it is to be understood that all proposed reaction conditions, including choice of solvent, reaction atmosphere, reaction temperature, duration of experiment and work-up procedures, are chosen to be conditions of standard for that reaction, which should be readily recognized by one skilled in the art. Not all compounds falling into a given class may be compatible with some of the reaction conditions required in some of the methods described. Such restrictions to the substituents which are compatible with the reaction conditions will be readily apparent to one skilled in the art and alternative methods can be used.

The compounds of the present invention or any intermediate could be purified, if required, using standard methods well known to a synthetic organist chemist, e.g. methods described in "Purification of Laboratory Chemicals", 6th ed. 2009, W. Amarego and C. Chai, Butterworth-Heinemann.

Starting materials are either known or commercially available compounds, or may be prepared by routine synthetic methods well known to a person skilled in the art.

### General Methods

Compounds of the invention may be prepared according to the following non-limiting general methods and examples:

The following abbreviations have been used throughout:
- AcOH: acetic acid
- Boc: tert-butoxycarbonyl
- CBz: benzyloxycarbonyl
- CDI: carbonyldiimidazole
- DEAD: diethyl azodicarboxylate
- DCC: dicyclohexylcarbodiimide
- DCM: dichloromethane
- DIAD: diisopropyl azodicarboxylate
- DIPEA: diisopropylethylamine
- DMF: *N,N*-dimethylformamide
- DMSO: dimethylsulfoxide
- dppf: 1,1'-bis(diphenylphosphino)ferrocene
- DTBPF: 1,1'-bis(di-tert-butylphosphino)ferrocene
- EDC: *N*-(3-Dimethylaminopropyl)-*N'*-ethylcarbodiimide
- EtOAc: ethyl acetate
- EtOH: ethanol
- HATU: 1-[Bis(dimethylamino)methylene]-1H-1,2,3-triazolo[4,5-b]pyridinium 3-oxide hexafluorophosphate
- HBTU: N,N,N',N'-Tetramethyl-O-(1H-benzotriazol-1-yl)uronium hexafluorophosphate
- LDA: lithium diisopropylamide
- Me: methyl
- MeCN: acetontitrile
- MeOH: methanol
- NBS: N-bromosuccinimide
- NCS: N-chlorosuccinimide
- NMP: N-methyl-2-pyrrolidinone
- SFC: supercritical fluid chromatography
- TEA: triethylamine
- TFA: trifluoroacetic acid
- THF: tetrahydrofuran
- T3P: propanephosphonic acid anhydride

### Scheme 1

Synthesis of compounds of general formula (I), wherein R₁, R₂ₐ, R_{2b} and R₃ are as previously defined. X is either H or CI, Hal is a suitable halogen, such as Br or I, and PG defines a suitable protecting group.

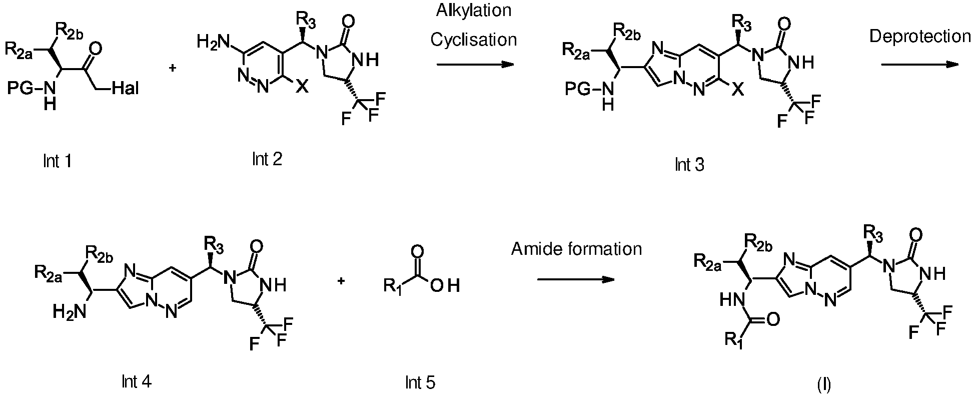

Compounds of general formula (I) can be prepared, as shown in Scheme 1. Compounds of formula (Int 1), which are synthesized, are reacted with compounds of general formula (Int 2) in a suitable solvent such as THF, in the presence of a suitable base such as sodium bicarbonate or DIPEA at between 65 and 80°C, and optionally in the presence of trimethylborate, to form compounds of general formula (Int 3). The protecting group can be removed by methods known to those skilled in the art, preferably when PG is Cbz, removal is effected in a suitable solvent such as MeOH or EtOH in the presence of an activated Palladium catalyst under a pressurized hydrogen atmosphere at ambient temperature, to form compounds of general formula (Int 4). Compounds of general formula (Int 5), which are either commercially available or are synthesized, are coupled with amines of general formula (Int 4), in the presence of a coupling reagent such as T3P, CDI, DCC, HATU, HBTU or EDC, and in the majority of cases, in the presence of a base, such as DIPEA or TEA, in a suitable solvent, such as DMF, THF or EtOAc to form compounds of formula (I).

### Scheme 2:

Synthesis of compounds of general formula (Int 12), wherein R₂ₐ and R_{2b} are as previously defined, and PG defines a suitable protecting group.

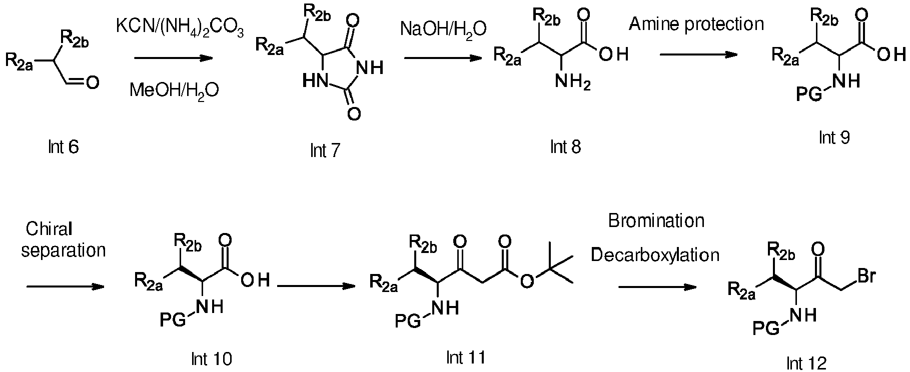

Compounds of formula (Int 12) can be prepared as shown in Scheme 2. Compounds of general formula (Int 6) which are either commercially available, or are synthesised, can be reacted with ammonium carbonate and potassium cyanide in water and methanol to form compounds of general formula (Int 7) (For Bucherer Bergs reaction, see: Chemical Reviews 2017 117 (23), 13757-13809). Compounds of general formula (Int 8) can be prepared by treatment of compounds of formula (Int 7) with alkali hydroxides such as sodium or potassium hydroxide in water. The amines of general formula (Int 8) can be protected by methods known to those skilled in the art, to give compounds of general formula (Int 9), where a suitable PG can be Boc or Cbz. Racemic compounds of general formula (Int 9) can be separated by chiral SFC, to give the S-enantiomers of compounds of general formula (Int 10, see WO2020127685A for methods). When R₂ₐ and R_{2b} together form 4-methylcyclohexyl, the amino acid of general formula (Int 10) can be synthesised as described in WO2018229079A1. tert-Butyl acetate is treated with a suitable organolithium, such as LDA, in THF at -78°C and reacted with the CDI activated products of compounds of general formula (Int 10), again at -78°C, to form compounds of general formula (Int 11). Halogenation with a suitable reagent such as NBS in a suitable solvent, such as MeOH, followed by decarboxylation, using TFA in an appropriate solvent, such as toluene at 80°C, affords compounds of general formula (Int 12).

### Scheme 3:

Synthesis of compounds of general formula (Int 21), wherein R₃ and X are H.

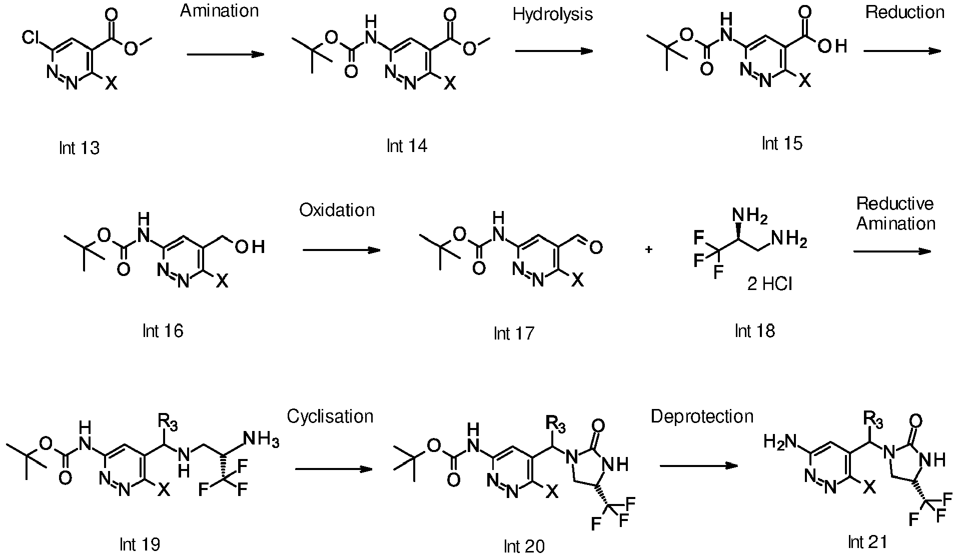

Compounds of formula (Int 21) can be prepared as shown in Scheme 3. To those skilled in the art, compounds of formula (Int 13) can be reacted using a variety of conditions to give compounds of formula (Int 14). Preferably using tert-butylcarbamate, in the presence of catalytic Pd₂(dba)₃ and a ligand such as DTBPF, or Pd(dppf).Cl₂, in toluene at 80°C. The compound of formula (Int 14) can be hydrolysed with sodium hydroxide in THF to form the compound of formula (Int 15). Those skilled in the art will appreciate the variety of methods for the production of compound of formula (Int 16) but preferably by formation of a mixed anhydride and subsequent reduction with sodium borohydride in MeOH at between -78°C and 0°C. Oxidation of the compound of formula (Int 16) with Dess-Martin periodinane in DCM at ambient temperature gives the compound of formula (Int 17). The compound of formula (Int 17) can react with (2S)-3,3,3-trifluoropropane-1,2-diamine;dihydrochloride (Int 18, see WO2020146194A for preparation), to form an imine that can be reduced with sodium cyanoborohydride in MeOH/AcOH to form the compound of formula (Int 19). Cyclisation to the compound of formula (Int 20) can be effected with CDI in THF at 65°C. Removal of the pivaloyl protecting group can be achieved by methods known to those skilled in the art to give the compound of formula (Int 21).

### Scheme 4:

Synthesis of compounds of general formula (Int 21), wherein R₃ is methoxymethyl and X is CI.

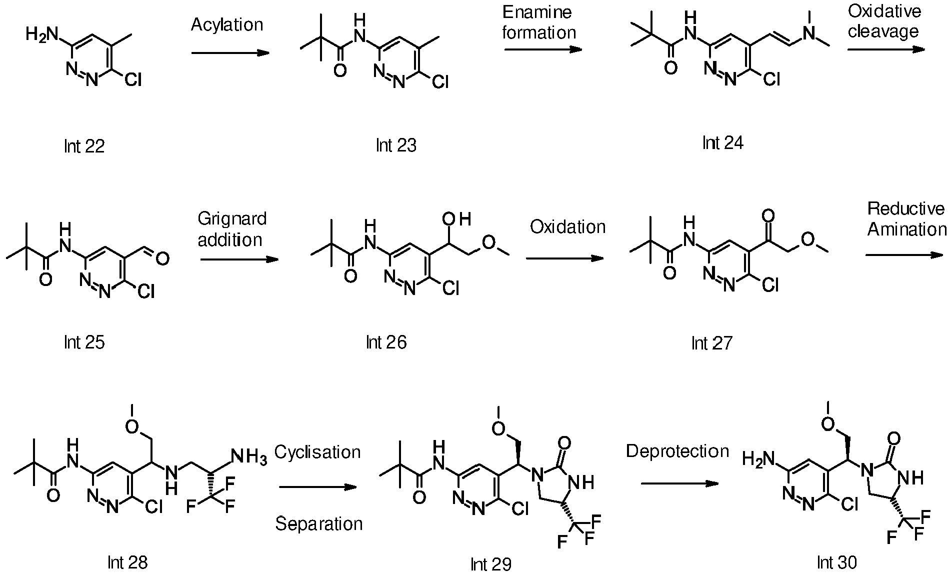

The compound of formula (Int 30) can be prepared as shown in Scheme 4. The amine of formula (Int 22) can be protected by methods known to those skilled in the art, preferably using pivaloyl chloride in a solvent such as NMP or DMF, with a suitable base such as pyridine. Reaction of the compound of formula (Int 23) with 1,1-diethoxy-N,N.dimethyl-methanamine in DMF at 120°C gives rise to the compound of formula (Int 24). Conversion of the enamine with sodium periodate in aqueous THF, affords the compound of formula (Int 25). The addition of a suitable Grignard reagent by methods known to those skilled in the art gives the compound of formula (Int 26). Oxidation of the compound of formula (Int 26) with Dess-Martin periodinane in DCM at ambient temperature gives compound of formula (Int 27). The compound of formula (Int 27) can react with (Int 18, see WO2020146194A for preparation), to form an imine that can be reduced with sodium cyanoborohydride in MeOH/AcOH to form the compound of formula (Int 28). Cyclisation to the compound of formula (Int 29) can be effected with CDI in THF at 65°C. The compound of formula (Int 30) can be prepared by the treatment of the compound of formula (Int 29) with aqueous HCl in MeOH at 100°C.

### Scheme 5:

Synthesis of compounds of general formula (Int 35), wherein R₃ is H and X is CI.

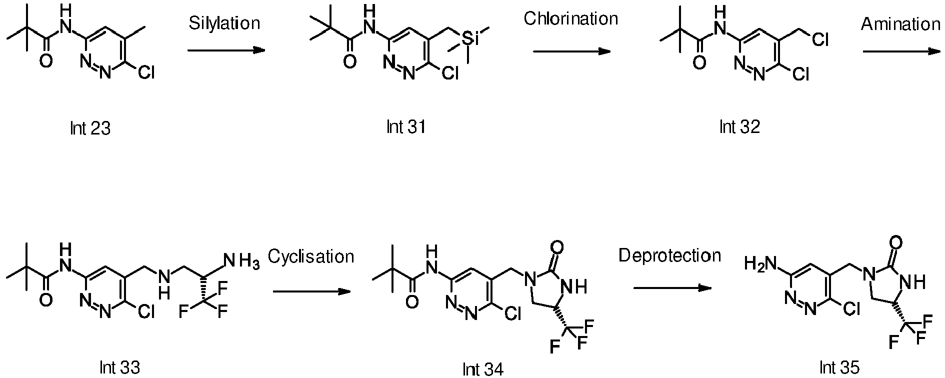

The compound of formula (Int 35) can be prepared as shown in Scheme 5. Treatment of the compound of formula (Int 23) with a suitable strong base such as n.butyllithium in THF at - 78°C with chlorotrimethylsilane will afford the compound of formula (Int 31). Chlorination of the compound of formula (Int 31) with NCS in DMF at ambient temperature affords the compound of formula (Int 32). The compound of formula (Int 33) can be accessed by the treatment of the compound of formula (Int 32) with (2S)-3,3,3-trifluoropropane-1,2-diamine;dihydrochloride in acetonitrile in the presence of sodium iodide and DIPEA. Cyclisation to the compound of formula (Int 34) is as for the formation of the compound of formula (Int 20). The compound of formula (Int 35) can be made by removal of the pivaloyl protecting group with aqueous hydrochloric acid at 70°C.

### Scheme 6:

Synthesis of compounds of general formula (Int 40), wherein Rₐ is as previously defined.

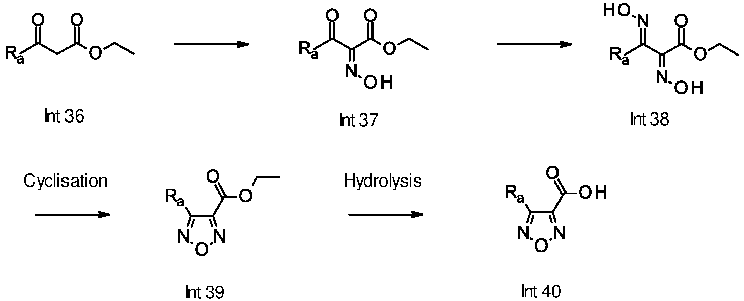

Compounds of formula (Int 40) can be prepared as shown in Scheme 6. Compounds of formula (Int 36), which are commercial or synthesised by literature methods, can react with aqueous sodium nitrate in a suitable solvent, such as AcOH, between 0°C and ambient temperature, to give compounds of general formula (Int 37). Treatment of compounds of formula (Int 37) with hydroxylamine hydrochloride in a suitable solvent such as EtOH, in the presence of either a suitable base such as sodium acetate or a suitable acid such as HCl in dioxane, between 60°C and 80°C, gives compounds of general formula (Int 38). Cyclisation to compounds of formula (Int 39) can be effected with CDI in THF. Acidic hydrolysis, using HCl in aqueous dioxane at 100°C or basic hydrolysis, using a suitable base such as LiOH or NaOH, in a suitable solvent such as aqueous THF or MeOH, affords compounds of formula (Int 40).

### Scheme 7:

Synthesis of compounds of general formula (Int 44), wherein Rₐ is as previously defined.

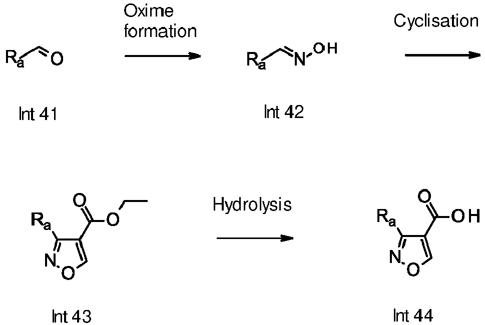

Compounds of general formula (Int 44) can be prepared as shown in Scheme 7. Compounds of formula (Int 41), which are commercial or synthesised by literature methods, can be treated by methods known to those skilled in the art, to give compounds of formula (Int 42). Compounds of formula (Int 42) can be halogenated with either NCS or NBS in DCM, then reacted with ethyl (E)-3-(dimethylamino)prop-2-enoate in a suitable solvent such as chloroform or DCM, in the presence of a suitable base, such as TEA, to give compounds of formula (Int 43). Compounds of formula (Int 44) can be prepared by basic hydrolysis of compounds of formula (Int 43) using LiOH or NaOH in a suitable solvent such as THF.

### Scheme 8

Preparation of compounds of formula (Int 47) wherein Rₐ is as previously defined.

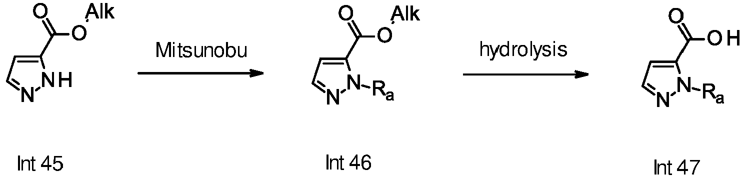

Compounds of general formula (Int 47) can be prepared as shown in Scheme 8 (see WO2020127685A for specific examples and preparations). Compound of formula (Int 45) that are commercial or synthesized can be reacted with alcohols, that are commercial or synthesized, under Mitsunobu conditions, namely in the presence of a phosphine such as triphenylphosphine and a diazodicarboxylate such as DEAD or DIAD, in a suitable solvent such as toluene or THF, to give compounds of formula (Int 46). Those skilled in the art will appreciate that some of the embodiments of Rₐ will undergo literature precedented transformations or deprotection, before hydrolysis with an appropriate base such as LiOH or NaOH in a suitable solvent such as MeOH or THF, to give compounds of general formula (Int 47).

### Scheme 9:

Preparation of compounds of formula (Int 50) wherein Rₐ is as previously defined.

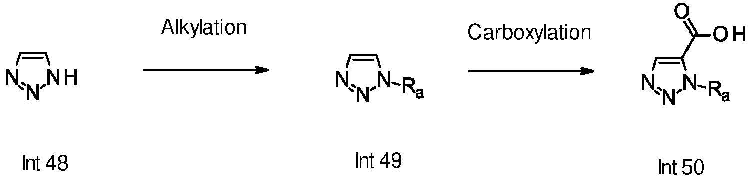

Compounds of general formula (Int 50) can be prepared as shown in Scheme 9. To those skilled in the art, the compound of formula (Int 48) can be alkylated to give compounds of formula (Int 49) that are not commercially available. Preferable conditions use an alkylhalide in a suitable solvent such as MeCN or DMF, in the presence of a suitable base such as potassium carbonate. Compounds of formula (Int 49) are treated with n-butyllithium in a suitable solvent such as THF at 0°C, in the presence of carbon dioxide, to give compounds of formula (Int 50). The activity of the compounds of the invention can be demonstrated in a suitable assay, such as the following.

### IL-8 release assay in human epithelial keratinocvtes adult (HEKa)

Keratinocytes were seeded at 3500 cells/well in 384-well ViewPlates (Perkin Elmer) in Epilife medium (Thermo Fisher) containing human keratinocyte growth supplement (HKGS) without hydrocortisone and incubated in a humid incubator at 37°C, 5% CO₂, overnight. The following day growth medium was removed and 25 µl fresh Epilife medium was added. 75 nL test compound in100% DMSO was added into each well reserved for test compounds, by the use of acoustic pipetting. The remaining wells received an equal volume of DMSO only, as vehicle control, or terfenadine in DMSO, as a positive control for any cytotoxic compounds. Subsequently, another 25 µL Epilife medium was added to each well. Finally, wells containing test compounds and wells prepared to yield maximum stimulation received 25 µL of 9 ng/mL recombinant, human embryonic kidney cell (HEK)-derived human IL-17AA + 30 ng/mL human TNF-alpha, in Epilife medium. Wells prepared to define 100% inhibition of IL-17 effects received 25 µL of 30 ng/mL human TNF-alpha alone, in Epilife medium. Final concentrations were 3 ng/mL HEK-human IL-17AA + 10 ng/mL human TNFalpha (maximum stimulation) and 10 ng/mL human TNFalpha alone (100% inhibition, Emax), respectively. Cells were incubated for 68-72 hours in the incubator. IL-8 released from the cells was measured by the use of a commercial homogenous time-resolved fluorescence (HTRF) assay (CisBio). 2 µL cell culture supernatant was transferred to a 384-well Proxiplate. 5 µL HTRF reagent was added and the plates were incubated sealed in the dark for 3-22 hours at room temperature. Time-resolved fluorescence was read at 665 vs 620 nm, with excitation at 320 nm, and IL-8 levels were calculated as percent of controls. Reduction of the amount of secreted IL-8 indicates decreased IL-17 signaling. Concentration response curves were fitted by the use of a four-parameter logistic equation. Relative IC₅₀ and Emax were reported from curves showing acceptable fit (r²>0.9). Cytotoxicity was measured in the cell-containing Viewplates following addition of 7 µL PrestoBlue (Thermo Fisher) and incubation for 2.5-3 hours at room temperature, by measuring fluorescence at 615 nm (excitation at 535 nm). Fluorescence was directly proportional to the amount of metabolic activity. Reduction of fluorescence signal indicated cytotoxicity.

### Embodiments:

Embodiment 1. A compound having the formula (I) 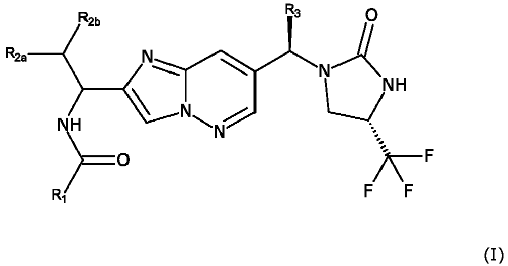
wherein R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from Rₐ;
Rₐ is (C₁-C₆)alkyl or (C₃-C₆)cycloalkyl, wherein said (C₁-C₆)alkyl and (C₃-C₆)cycloalkyl, is optionally substituted with one or more substituents independently selected from deuterium, halogen, hydroxy, and cyano;
R₂ₐ and R_{2b} are independently cyclopropyl and cyclobutyl or R₂ₐ and R_{2b} together form a 4-methyl-cyclohexyl ring;
R₃ is hydrogen or methoxymethyl;
or pharmaceutically acceptable salts, hydrates and solvates thereof.

Embodiment 2. The compound according to embodiment 1 having the formula (Ia) 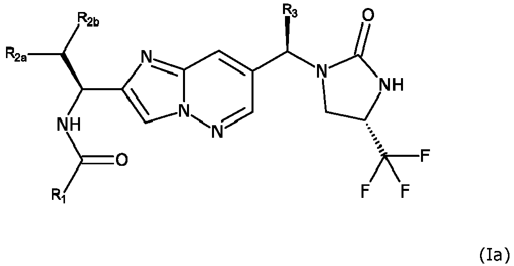 wherein R₁, R₂ₐ, R_{2b} and R₃ are as defined in claim 1 or pharmaceutically acceptable salts, hydrates and solvates thereof.

Embodiment 3. The compound according to any one of embodiments 1-2, wherein R₁ is substituted with one Rₐ and Rₐ is selected from (C₁-C₆)alkyl and (C₃-C₆)cycloalkyl, wherein said (C₁-C₆)alkyl or (C₃-C₆)cycloalkyl, is optionally substituted with one or more substituents independently selected from deuterium, fluoro, hydroxy, and cyano.

Embodiment 4. The compound according to any one of embodiments 1-3, wherein
R₂ₐ and R_{2b} are both cyclopropyl and
R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from (C₁-C₆)alkyl and (C₃-C₆)cycloalkyl and fluoro(Ci-C₆)alkyl.

Embodiment 5. The compound according to embodiment 4, wherein the 1,2,3-triazole, pyrazole, isoxazole and oxadiazole is substituted with one substituent selected from methyl, ethyl, isopropyl, cyclopropyl, difluromethyl and trifluoromethyl.

Embodiment 6. The compound according to any one of embodiments 1-2, wherein
R₂ₐ and R_{2b} are both cyclopropyl and R₁ is 1,2,3-triazole optionally substituted with one or more substituents independently selected from Rₐ.

Embodiment 7. The compound according to any one of embodiments 1-2, wherein
R₂ₐ and R_{2b} are both cyclopropyl and R₁ is pyrazole optionally substituted with one or more substituents independently selected from Rₐ.

Embodiment 8. The compound according to any one of embodiments 1-2, wherein
R₂ₐ and R_{2b} are both cyclopropyl and R₁ is isoxazole optionally substituted with one or more substituents independently selected from Rₐ.

Embodiment 9. The compound according to any one of embodiments 1-2, wherein
R₂ₐ and R_{2b} are both cyclopropyl and R₁ is oxadiazole optionally substituted with one or more substituents independently selected from Rₐ.

Embodiment 10. The compound according to embodiment 7, wherein
R₂ₐ and R_{2b} are both cyclopropyl and R₁ is pyrazol-3-yl optionally substituted with one or more substituents independently selected from Rₐ.

Embodiment 11. The compound according to embodiment 10, wherein R₁ is pyrazol-3-yl substituted in position 2 with (C₁-C₆)alkyl, (C₃-C₆)cycloalkyl or fluoro(C₁-C₆)alkyl Embodiment 12. The compound according to embodiment 11, wherein R₁ is pyrazol-3-yl substituted in position 2 with ethyl, isopropyl, cyclopropyl, difluoromethyl or trifluoromethyl.

Embodiment 13. The compound according to any one of embodiments 1-3, wherein
R₂ₐ and R_{2b} together form 4-methyl-cyclohexyl; and
R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from (C₁-C₆)alkyl, (C₃-C₆)cycloalkyl and fluoro(C₁-C₆)alkyl.

Embodiment 14. The compound according to embodiment 13, wherein the 1,2,3-triazole, pyrazole, isoxazole and oxadiazole is substituted with one substituent selected from methyl, ethyl, isopropyl, cyclopropyl, difluoromethyl and trifluoromethyl.

Embodiment 15. The compound according to any one of embodiments 1-2, wherein
R₂ₐ and R_{2b} together form 4-methyl-cyclohexyl and R₁ is 1,2,3-triazole optionally substituted with one or more substituents independently selected from Rₐ.

Embodiment 16. The compound according to any one of embodiments 1-2, wherein
R₂ₐ and R_{2b} together form 4-methyl-cyclohexyl and R₁ is pyrazole optionally substituted with one or more substituents independently selected from Rₐ.

Embodiment 17. The compound according to any one of embodiments 1-2, wherein
R₂ₐ and R_{2b} together form 4-methyl-cyclohexyl and R₁ is isoxazole optionally substituted with one or more substituents independently selected from Rₐ.

Embodiment 18. The compound according to any one of embodiments 1-2, wherein
R₂ₐ and R_{2b} together form 4-methyl-cyclohexyl and R₁ is oxadiazole optionally substituted with one or more substituents independently selected from Rₐ.

Embodiment 19. The compound according to embodiment 16, wherein
R₂ₐ and R_{2b} together form 4-methyl-cyclohexyl and R₁ is pyrazol-3-yl optionally substituted with one or more substituents independently selected from Rₐ.

Embodiment 20. The compound according to embodiment 19, wherein R₁ is pyrazol-3-yl substituted in position 2 with (C₁-C₆)alkyl, (C₃-C₆)cycloalkyl and fluoro(C₁-C₆)alkyl.

Embodiment 21. The compound according to embodiment 20, wherein R₁ is pyrazol-3-yl substituted in position 2 with ethyl, isopropyl, cyclopropyl, difluoromethyl and trifluoromethyl.

Embodiment 22. The compound according to any one of embodiments 1-21, wherein R₁ carries one substituent Rₐ and Rₐ is (C₁-C₆)alkyl or (C₃-C₆)cycloalkyl.

Embodiment 22. The compound according to embodiment 23, wherein Rₐ is ethyl, isopropyl or cyclopropyl.

Embodiment 23. The compound according to any one of embodiment 1-22, wherein R₃ is methoxymethyl.

Embodiment 24. The compound according to any one of embodiments 1-22, wherein R₃ is hydrogen.

## Claims

1. A compound having the formula (I) 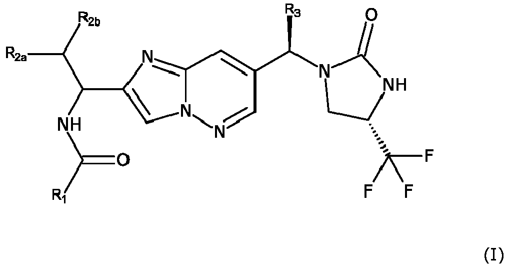
wherein R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from Rₐ;
Rₐ is (C₁-C₆)alkyl or (C₃-C₆)cycloalkyl, wherein said (C₁-C₆)alkyl and (C₃-C₆)cycloalkyl, is optionally substituted with one or more substituents independently selected from deuterium, halogen, hydroxy, and cyano;
R₂ₐ and R_{2b} are independently cyclopropyl and cyclobutyl or R₂ₐ and R_{2b} together form a 4-methyl-cyclohexyl ring;
R₃ is hydrogen or methoxymethyl;
or pharmaceutically acceptable salts, hydrates and solvates thereof.

2. The compound according to claim 1 having the formula (Ia) 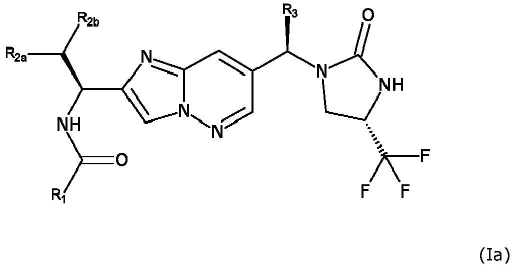 wherein R₁, R₂ₐ, R_{2b} and R₃ are as defined in claim 1 or pharmaceutically acceptable salts, hydrates and solvates thereof.

3. The compound according to any one of claims 1-2, wherein R₂ₐ and R_{2b} are both cyclopropyl.

4. The compound according to any one of claims 1-2, wherein R₂ₐ and R_{2b} together form 4-methyl-cyclohexyl.

5. The compound according to any one of claims 1-4, wherein
R₁ is selected from 1,2,3-triazole, pyrazole, isoxazole and oxadiazole, wherein the 1,2,3-triazole, pyrazole, isoxazole and oxadiazole may optionally be substituted with one or more substituents independently selected from (C₁-C₆)alkyl, (C₃-C₇)cycloalkyl and fluoro(C₁-C₆)alkyl.

6. The compound according to any one of claims 1-5, wherein R₁ is optionally substituted 1,2,3-triazole.

7. The compound according to any one of claims 1-5, wherein R₁ is optionally substituted pyrazole.

8. The compound according to any one of claims 1-5, wherein R₁ is optionally substituted isoxazole.

9. The compound according to any one of claims 1-5, wherein R₁ is optionally substituted oxadiazole.

10. The compound according to claim 1 selected from
N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-4-methyl-1,2,5-oxadiazole-3-carboxamide;
4-cyclopropyl-N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-1,2,5-oxadiazole-3-carboxamide;
N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-2-isopropyl-pyrazole-3-carboxamide;
N-[(1S)-2,2-dicyclopropyl-1-[7-[(1S)-2-methoxy-1-[(4S)-2-oxo-4-(trifluoromethyl)imidazolidin-1-yl]ethyl]imidazo[1,2-b]pyridazin-2-yl]ethyl]-3-ethyl-isoxazole-4-carboxamide;
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-4-methyl-1,2,5-oxadiazole-3-carboxamide;
4-cyclopropyl-N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-1,2,5-oxadiazole-3-carboxamide;
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-2-isopropyl-pyrazole-3-carboxamide;
N-[(1S)-2,2-dicyclopropyl-1-[7-[[(4S)-2-oxo-4-(trifluoromethyl)imidazolidin-1-yl]methyl]imidazo[1,2-b]pyridazin-2-yl]ethyl]-3-ethyl-isoxazole-4-carboxamide;
N-[(S)-[7-[(1S)-2-methoxy-1-[(4S)-2-oxo-4-(trifluoromethyl)imidazolidin-1-yl]ethyl]imidazo[1,2-b]pyridazin-2-yl]-(4-methylcyclohexyl)methyl]-4-methyl-1,2,5-oxadiazole-3-carboxamide; and
4-methyl-N-[(S)-(4-methylcyclohexyl)-[7-[[(4S)-2-oxo-4-(trifluoromethyl)imidazolidin-1-yl]methyl]imidazo[1,2-b]pyridazin-2-yl]methyl]-1,2,5-oxadiazole-3-carboxamide or pharmaceutically acceptable salts, hydrates and solvates thereof.

11. A compound according to any one of claims 1-10 for use in therapy.

12. A compound according to claim 11 for use in treatment of a disease, disorder or condition, which disease, disorder or condition is responsive of modulation of IL-17.

13. A compound according to claim 11 for use in treatment of autoimmune diseases.

14. A compound according to claim 11 for use in treatment of psoriasis, ankylosing spondylitis, spondyloarthritis or psoriatic arthritis.

15. A pharmaceutical composition comprising a compound according to any one of claims 1-10 together with a pharmaceutically acceptable vehicle or excipient or pharmaceutically acceptable carrier(s).

16. The pharmaceutical composition according to claim 15 together with one or more other therapeutically active compound(s).
